# DEMANDE DE BREVET EUROPEEN

(11) **EP 0 545 759 A1**
(43) Date de publication de la demande: **09.06.1993**
(21) Numéro de dépôt: 92403120.6
(22) Date de dépôt: 20.11.1992
(51) Int. Cl.: A61K 31/195, A61K 31/44

(54) **Utilisation d'un acide aminé soufré, éventuellement en association avec la pyridoxine, pour la préparation d'un médicament destiné au traitement de l'eczéma**

(30) Priorité: 28.11.1991 FR 9114741
(71) Demandeur: Société BFB, F-93360 Neuilly Plaisance (FR)
(72) Inventeur: Bigou, Alphonse, F-75116 Paris (FR)
(74) Mandataire: Hubert, Philippe

(57) **Abrégé**

La présente invention concerne généralement L'utilisation d'un acide aminé soufré, de préférence choisi parmi la cystine, la cystéine, et la méthionine, éventuellement en combinaison avec la pyridoxine, pour la préparation d'un médicament destiné au traitement de l'eczéma, de préférence de l'eczéma de contact.

## Description

La présente invention concerne généralement une nouvelle indication thérapeutique de produits connus, et a essentiellement pour objet l'utilisation d'un acide aminé soufré pour la préparation d'un médicament destiné au traitement de l'eczéma, de préférence de l'eczéma de contact.

On sait que parmi les traitements proposés jusqu'à ce jour pour soigner l'eczéma, le traitement par la cortisone est le plus communément prescrit.

Cependant, la cortisone présente de nombreux effets secondaires indésirables, de sorte que son utilisation est souvent réservée aux cas les plus sérieux d'eczéma.

Il a été découvert, et ceci constitue le fondement de la présente invention, que les acides aminés soufrés présentent une très grande efficacité, notamment lorsqu'ils sont administrés par voie orale, pour le traitement de l'eczéma, notamment l'eczéma de contact, et que ces produits ne présentent aucun effet secondaire indésirable.

Ainsi, la présente invention vise généralement à couvrir l'utilisation d'un acide aminé soufré pour la préparation d'un médicament ou d'une composition pharmaceutique destinée au traitement de l'eczéma, de préférence de l'eczéma de contact.

Selon une caractéristique particulière de l'invention, l'acide aminé soufré est choisi parmi la cystine, la cystéine et la méthionine.

Ces acides aminés soufrés connus ont reçu un certain nombre d'applications, soit en dermatologie, dans le traitement des affections phanériennes, soit en ophtalmologie en tant qu'agents anticollagénasiques, et leur activité thérapeutique, dans le traitement de l'eczéma est tout à fait inattendu.

Selon un mode de réalisation actuellement préféré de l'invention, l'acide aminé soufré sera utilisé en association avec la pyridoxine (connue sous la dénomination Vitamine B6).

Les propriétés toxicologiques de ces produits sont parfaitement connues. On sait par exemple que ces acides aminés soufrés ne présentent aucune toxicité, que la pyridoxine peut être absorbée en quantité relativement importante sans montrer d'actions secondaires nocives marquées.

Un mélange d'acide aminé soufré et de pyridoxine selon l'invention est donc non-toxique.

Lorsque l'acide aminé soufré est utilisé en combinaison avec la pyridoxine, un médicament conforme à l'invention comportera de 5 à 15 parties en poids d'acide aminé pour une partie en poids de pyridoxine.

Un médicament préparé conformément à la présente invention sera présenté de préférence sous forme de comprimés simples ou dragéifiés, de gélules, de granulés, et plus généralement sous toute forme utilisable par voie orale. Bien évidemment, ces médicaments pourront comporter les excipients pharmaceutiquement acceptables employés habituellement, comme par exemple le talc, l'amidon, la carboxyméthylcellulose, ou un sucre.

La posologie variera notamment en fonction de la voie d'administration, de l'affection traitée et du sujet en cause.

D'une façon générale, un médicament conforme à l'invention sera préparé sous forme de comprimé dosé de 200 à 1000 mg.

Dans le traitement de l'eczéma de contact, un adulte de poids moyen pourra absorber par exemple 500 mg d'acide aminé soufré par dose, ladite dose pouvant être renouvelée de 3 à 8 fois par jour, si nécessaire.

Les exemples non limitatifs suivants permettront d'illustrer la présente invention.

On a réalisé des comprimés dragéifiés de cystine et de pyridoxine répondant aux formules suivantes :

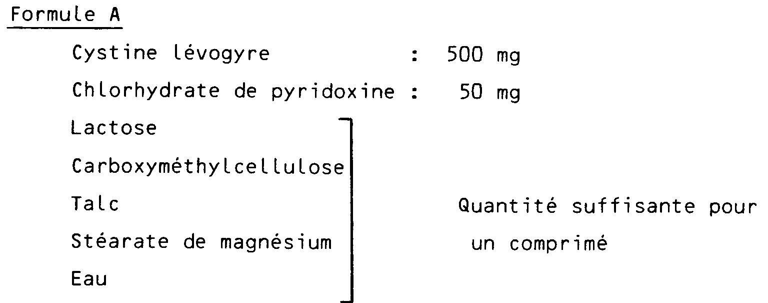

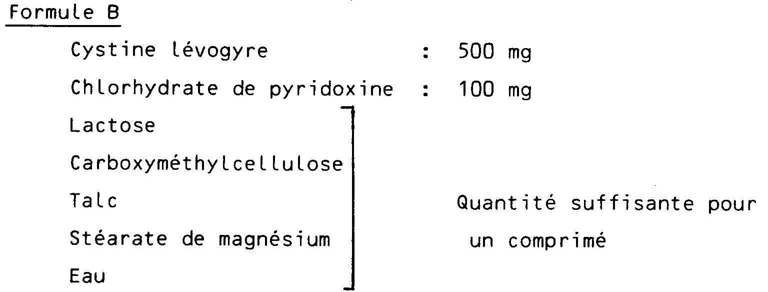

Le médicament ainsi préparé (formule B) a été administré à raison de 3 à 8 comprimés par jour à divers malades souffrant d'eczéma chronique. Ce traitement a entraîné de façon tout à fait surprenante une rémission importante et voir même dans certains cas une rémission complète et définitive.

On a en particulier réalisé une étude clinique sur deux groupes de patients présentant respectivement une allergie au nickel et une allergie aux baumes du Pérou.

Les patients du premier groupe présentaient un érythème prurigineux avec vésicules et/ou lichénification des lésions, et un test positif au nickel.

Les patients du second groupe présentaient également un érythéme prurigineux avec vésicules et/ou lichénification des lésions et un test positif aux Baumes du Pérou.

L'efficacité du traitement a été appréciée par un critère allergologique (évolution de la positivté aux tests, au nickel ou aux baumes du Pérou, après 50 jours de suivi) et par des critères cliniques (évolution des symptômes cliniques tels qu'étendue et aspect de l'érythème, des vésicules, diminution ou disparition du prurit, du suintement, etc.).

Sur l'ensemble des patients traités, on a observé dans environ 75 % des cas une amélioration très nette et dans certains cas une rémission complète et définitive.

Des essais complémentaires ont montré que l'efficacité de ce traitement est essentiellement due à la cystine, et plus généralement à tout acide aminé soufré de préférence choisi parmi la méthionine, la cystéine et la cystine.

La pyridoxine exerce dans cette application un effet très net de potentialisation de l'action des acides aminés soufrés précités.

## Revendications

1. Utilisation d'un acide aminé soufré pour la préparation d'un médicament destiné au traitement de l'eczéma, de préférence de l'eczéma de contact.

2. Utilisation selon la revendication 1, caractérisée en ce que l'acide aminé soufré précité est choisi parmi la cystine, la cystéine et la méthionine.

3. Utilisation selon la revendication 1 ou 2, caractérisée en ce que l'acide aminé soufré précité est utilisé en association avec la pyridoxine.

4. Utilisation selon l'une des revendications 1 à 3, caractérisée en ce que le médicament est préparé sous toute forme utilisable par voie orale, de préférence sous forme de comprimés dosés de 200 à 1000 mg.

5. Utilisation selon les revendications 3 et 4, caractérisée en ce que le médicament comporte de 5 à 15 parties en poids d'acide aminé soufré pour une partie en poids de pyridoxine.

6. Utilisation selon la revendication 5, caractérisée en ce que l'acide aminé soufré est la cystine.

7. Utilisation selon la revendication 6, pour la préparation d'un médicament comportant 500 mg de cystine et 50 mg de pyridoxine.

8. Utilisation selon la revendication 6, pour la préparation d'un médicament comportant 500 mg de cystine et 100 mg de pyridoxine.
